Europäisches Patentamt

European Patent Office

Office européen des brevets

(19)

(11) Publication number: **0 080 277**
**A2**

# EUROPEAN PATENT APPLICATION

(12)

(21) Application number: **82305793.0**

(22) Date of filing: **01.11.82**

(51) Int. Cl.³: **C 12 N 1/00**

(30) Priority: **23.11.81 GB 8135236**

(43) Date of publication of application: **01.06.83**
**Bulletin 83/22**

(84) Designated Contracting States: **DE FR GB**

(71) Applicant: **IMPERIAL CHEMICAL INDUSTRIES PLC, Imperial Chemical House Millbank, London SW1P 3JF (GB)**

(72) Inventor: **Bell, Kenneth Scott, 26 Birkdale Road Hartburn, Stockton-on-Tees Cleveland (GB)**
Inventor: **Douglas, Arthur Jenner, 24 Tintern Avenue, Billingham Cleveland (GB)**
Inventor: **Sherrington, Peter John, Dr., Stillington Moor House Nr Bishopton, Stockton-on-Tees Cleveland (GB)**

(74) Representative: **Aufflick, James Neil et al, Imperial Chemical Industries PLC Legal Department: Patents Thames House North Millbank, London SW1P 4QG (GB)**

(54) **Apparatus for continuously granulating and drying single cell protein.**

(57) Apparatus for the continuous granulation and drying of single cell protein which comprises a plurality of components in combination including a granulator, gas heating means and a cyclone. The components are connected in such a manner that a stream of wet single cell protein product from a fermenter can pass into the granulator and from the granulator into a heated gas stream which carries it to a cyclone. After leaving the cyclone a proportion of the granulated product is returned to the granulator.

EP 0 080 277 A2

Apparatus for continuously granulating and drying single
cell protein

This invention relates to an apparatus for the continuous
granulation and drying of a single cell protein product and in
particular to an apparatus useful for the continuous granulation
and drying of single cell protein produced by cultivation of a
strain of the species Methylophilus methylotrophus.

In recent years considerable attention has been paid to
the development of processes for the production of single cell
protein by growing microorganisms upon culture media containing
as carbon sources hydrocarbons and oxygenated hydrocarbons, particularly alcohols. Mainly this attention has been directed towards the isolation of suitable microorganisms, the optimisation
of culture conditions and the development of fermenters suitable
for the commercial production of single cell protein on a large
scale. However in the development of a successful commercial
process for the production of single cell protein the treatment
of the microorganisms after cultivation is considerably important.
Hitherto the attention paid to post-cultivation treatment of microorganisms has been comparatively less than the attention paid to,
e.g. optimisation of culture conditions and development of suitable fermenters. It is very important that the end product of a
process for the production of single cell protein should be dry,
granular and substantially non-dusty.

According to the present invention we provide an apparatus for the continuous granulation and drying of single cell protein which comprises in combination the following components:-

(a)       a granulator;

(b)       connecting means for connecting the granulator to an apparatus for the production of single cell protein;

(c)       gas heating means;   and

(d)       one or more cyclones,

the components being connected together in such a manner that a wet single cell protein product passes continuously through the connecting means into the granulator, granulated product passes continuously into a heated gas stream from the gas heating means and is carried by the stream into the cyclone, a proportion of the gas leaving the cyclone being returned to the gas heating means and a proportion of the granulated product leaving the cyclone being returned to the granulator.

In an apparatus for the production of single cell protein a culture of microorganisms is grown continuously in a fermenter to which nutrient medium is supplied continuously and from which product culture is withdrawn continuously.  The withdrawn culture passes through a series of post-culturing treatment steps before being treated by the granulation and drying apparatus of the invention.  In a typical apparatus for the production of single cell protein the post-cultivation treatment steps preceding granulation and drying comprise in sequence a heating step, a cooling step, an acidification step, a flotation separation step and a centrifugation step.  From the centrifugation step a thick suspension or "cream" containing microorganism cells passes to the granulator of the apparatus of the present invention.  In the single cell protein production apparatus, the heating, cooling and acidification steps constitute a harvesting treatment according to our UK Patent Specification No. 1381306 (corresponding to US Patent 3878093) which produces flocculation of the microorganism cells.  Variants of the post-cultivation treatment steps are possible.  For instance in the harvesting treatment the acidification step may precede the heating step.  Flotation separation may be replaced by sedimentation separation, in which case a degasification step is suitably included between the harvesting treatment and the separation step. Preferably the fermenter used in the process for the production of single cell protein is an air-lift or "pressure-cycle" fermenter. Suitable fermenters are described in our UK Patent Specifications Nos. 1353008 (corresponding to US Patent No. 3847748), 1417486, 1417487, 1525930 (corresponding to US Patent 4183787) and 2002417B (corresponding to US Patent 4237693).

The granulation and drying apparatus of the present invention is very suitable for use in carrying out the process of our UK Patent Specification No. 1370892 (corresponding to US Patent 3989594) and particularly when this process is used for the cultivation of a strain of the species Methylophilus methylotrophus (formerly named Pseudomonas methylotropha) in a culture medium containing methanol as the carbon source. Representative strains of the species Methylophilus methylotrophus have been deposited at the National Collection of Industrial Bacteria (NCIB), Torrey Research Station, Aberdeen, Scotland, UK where they have been assigned the following accession numbers:- NCIB 10508-15 and 10592-6. Equivalent cultures have also been deposited at the NRRL Culture Collection maintained by the US Department of Agriculture at Peoria, Illinois, USA where they have been assigned the following accession numbers:- NRRL B5352-5364. Strains of this species are particularly suitable for granulation using the granulation and drying apparatus of the present invention.

During use of the apparatus of the present invention, a thick suspension of microorganisms or "cream" passes from a centrifuge into a granulator which already contains granules of the single cell protein. Suitable granulators are of the ploughshare mixer type. Granulated single cell protein passes into the drying loop and is entrained in a hot gas leaving a furnace at a temperature in the range $200^{\circ}C$ to $500^{\circ}C$, depending upon the feed rate of cream and normally being about $500^{\circ}C$ at high feed rates. In the gas stream the granules are flash dried and pass one or more cyclones, a plurality of cyclones being referred to as a bank of cyclones. Suitable cyclones are long cone, high efficiency cyclones. From the cyclone a large proportion, ranging between 10 and 15 times the production rate of the granules, are returned to the granulator to maintain the desired moisture level of granules therein. The remainder of the granules passing from the cyclone form the product of the process for producing single cell protein. From the cyclone the gas used to entrain the granules is re-cycled to the furnace to be re-heated, passing through a dust scrubber and condenser during re-cycling. A proportion of the gas is purged from the gas stream in order that its oxygen concentration may be kept at a level (suitably 4% to 8% and preferably about 8%) which is below that at which dust explosions can occur at the operating temperatures. Gas purged from the drying loop is scrubbed to eliminate organic odour before being discharged into the atmosphere.

The closed loop granulation and drying system of the invention is advantageous in that it prevents dust escaping to the atmosphere to the detriment of the environment. The properties of strains of the species <u>Methylophilus</u> <u>methylotrophus</u> render these strains particularly advantageous for use in the apparatus of the invention.

The invention is illustrated in the accompanying drawings wherein:-

Figure 1 is a schematic diagram of one form of a plant for the production of single cell protein.

Figure 2 is a schematic diagram of an apparatus for continuous granulation and drying of single cell protein according to the present invention.

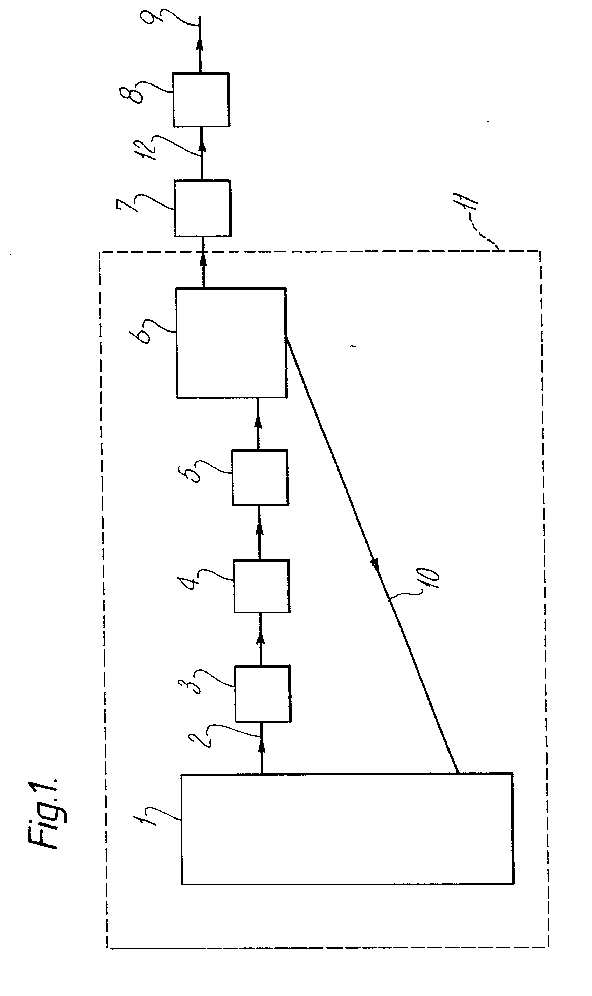

The plant shown in Figure 1 comprises a fermenter 1, pipe means 2 for withdrawing culture from the fermenter, heating means 3, cooling means 4, acidification means 5, flotation separation means 6, centrifuge 7, granulation and drying loop 8, product removal chute 9 and recycle pipe 10. Pipe 12 conveys wet single protein product from centrifuge 7 to the granulator in granulation and drying loop 8. Fermenter 1, heating means 3, cooling means 4, acidification means 5, flotation separation means 6 and recycle pipe 10 are all included in a sterile area all entries to and exits from which are provided with sterilization means to prevent viable microorganisms from entering the sterile area. The notional boundary of the sterile area is shown by broken line 11.

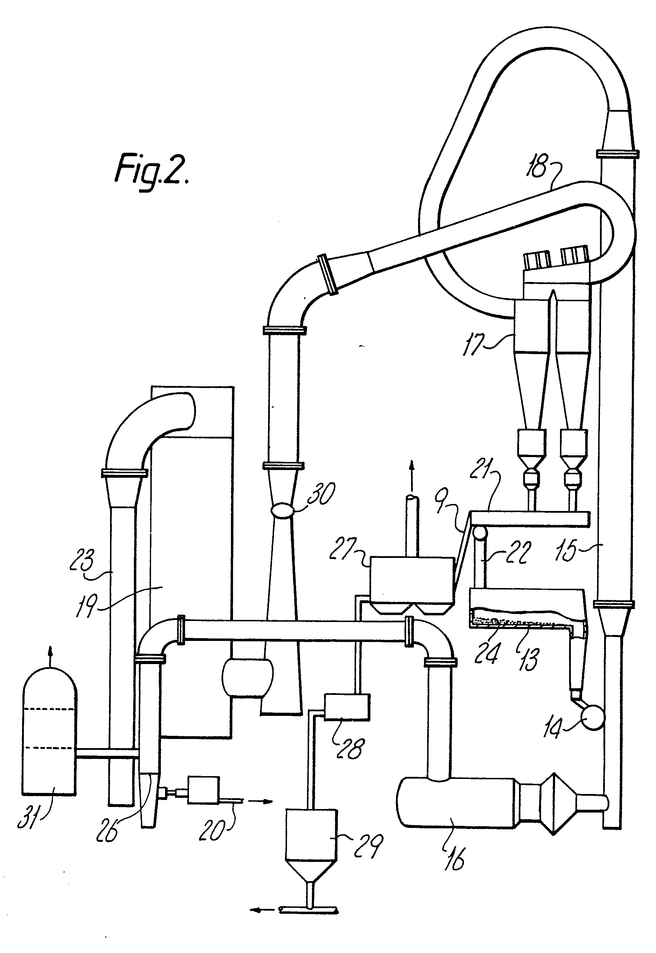

Figure 2 shows in detail granulation and drying loop 8, i.e. the apparatus of the present invention. Pipe 12 conveys wet single cell protein product from centrifuge 7 (shown in Figure 1) into granulator 13 which contains a bed of granules 24. From granulator 13 granules are fed by transporting mechanism 14 into drier tube 15 in which they are entrained by hot gas from furnace 16. A mixture of fresh air and gas (not shown in drawing) is blown into furnace 16 and the combustion products pass into drier tube 15 at a temperature of up to $500^{o}$C. Hot gas with entrained granular single cell protein passes up drier tube 15 into cyclone 17 (of which a plurality are employed). On entry into cyclone 17 the hot gas is at a temperature in the range $105^{o}$ to $135^{o}$C. Dried granular single cell protein product leaves cyclone 17 along collecting screw conveyors 21 and is divided into two portions. One portion which is the final product of the single cell protein production process passes along product removal chute 9 to be cooled and classified in product cooler 27, oiled in oiling mixer 28 and then transferred to storage bunker (not shown in drawing) via feed hopper 29. The major portion passes along chute 22 into granulator 13. Gas leaves cyclone 17 along pipe 18 and passes to dust scrubber 30 and condenser 19. It is then re-cycled along pipe 23 using fan 20 to furnace 16. The oxygen concentration of the gas in furnace 16 is maintained at a proportion below 8% to avoid dust explosions, to maintain this proportion part of the gas being re-cycled to the furnace is purged from the re-cycle loop at 26. The purged gas is scrubbed by a proprietory device 31 to remove any residual dust before being exhausted to the atmosphere.

In the plant shown in Figure 1 fresh nutrients are added to fermenter 1 continuously either directly or via re-cycle pipe 10 by means not shown in Figure 1

PA/JNA/MP
4 October 1982

1.        An apparatus for the continuous granulation and drying of single cell protein which comprises in combination the following components:-

(a)        a granulator;

(b)        connecting means for connecting the granulator to an apparatus for the production of single cell protein;

(c)        gas heating means;   and

(d)        one or more cyclones,

the components being connected together in such a manner that a wet single cell protein product passes continuously through the connecting means into the granulator, granulated product passes continuously into a heated gas stream from the gas heating means and is carried by the stream into the cyclone, a proportion of the gas leaving the cyclone being returned to the gas heating means and a proportion of the granulated product leaving the cyclone being returned to the granulator.

2.        An apparatus according to claim 1 wherein the temperature of the gas in the stream leaving the gas heating means is within the range $200^{\circ}C$ to $500^{\circ}C$.

3.        An apparatus according to claim 1 or claim 2 wherein a proportion ranging between 10 and 15 times the production rate of the granules are returned to the granulator to maintain the desired moisture level of granules therein.

4.        An apparatus according to any one of claims 1 to 3 wherein the gas stream leaving the cyclone passes to a condenser and thereafter part of the gas stream is purged to the atmosphere.

5.        An apparatus for the continuous granulation and drying of single cell protein substantially as described herein and as shown in Figure 2 of the drawings.

0080277

Fig.1.

0080277

Fig.2.